# EUROPEAN PATENT SPECIFICATION

(11) **EP 4 248 850 B1**
(45) Date of publication and mention of the grant of the patent: **26.08.2026**
(21) Application number: 23161408.2
(22) Date of filing: 13.03.2023
(51) Int. Cl.: A61B 5/021, A61B 5/022

(54) **APPARATUS AND METHODS FOR TRIGGERING BLOOD PRESSURE MEASUREMENTS**
VORRICHTUNG UND VERFAHREN ZUR AUSLÖSUNG VON BLUTDRUCKMESSUNGEN
APPAREIL ET PROCÉDÉS POUR DÉCLENCHER DES MESURES DE PRESSION SANGUINE

(30) Priority: 25.03.2022 US 202217656511
(43) Date of publication of application: 27.09.2023
(73) Proprietor: GE Precision Healthcare LLC, Wauwatosa, WI 53226 (US)
(72) Inventor: VIRTANEN, Juha, Wauwatosa, 53226 (US); COFFENG, René, Wauwatosa, 53226 (US); TAKALA, Panu, Wauwatosa, 53226 (US); HELLMAN, Emma, Wauwatosa, 53226 (US)
(74) Representative: Fennell, Gareth Charles

(56) References cited:
- US-A- 5 785 659
- US-A1- 2006 041 281
- US-A1- 2011 054 328
- US-A1- 2011 144 456
- US-A1- 2014 121 546
- US-A1- 2015 099 953

## Description

### TECHNICAL FIELD

This disclosure relates generally to blood pressure measurement devices, and more specifically, to blood pressure measurement devices that are configured to take blood pressure measurements at specified times.

### BACKGROUND

Non-invasive blood pressure measuring devices are frequently used to measure the blood pressure of an individual, such as a patient in a hospital or doctor's office. For example, inflatable blood pressure cuffs may be used to measure the blood pressure of a patient. In some cases, it may be helpful to measure a blood pressure of a patient frequently. However, in some cases, it may be detrimental to measure a blood pressure of a patient too frequently. For example, in some cases, it may be painful or harmful to an arm of a patient to measure a blood pressure too frequently. US2011/054328 A1 describes a blood pressure measurement system.

### SUMMARY

The current invention is defined by the appended claims. In a general aspect, an apparatus comprising a monitor, a blood pressure measuring device, and a controller. The monitor is configured to estimate a blood pressure of a patient. The blood pressure measuring device is configured to measure a blood pressure of the patient. The controller is operatively coupled to the monitor and to the blood pressure measuring device. The controller is configured to cause the blood pressure measuring device to take a first blood pressure measurement of the patient in response to receiving an estimated blood pressure deviating from a threshold from the monitor. The controller is also configured to prevent the blood pressure measuring device from taking a second blood pressure measurement of the patient until a time period has expired since the first blood pressure measurement of the patient.

In another aspect, an apparatus, comprising an input, an output, and a controller. The input is configured to receive a signal indicative of a blood pressure estimate of a patient. The output is configured to provide a signal to take a first blood pressure measurement of the patient in response receiving a signal indicating that an estimated blood pressure of the patient deviates from a baseline. The controller is coupled to the input and to the output and is configured to prevent a second blood pressure measurement of the patient until a time period has expired since the first blood pressure measurement of the patient.

In another aspect, a non-transitory computer-readable medium including instructions that, when executed, are configured to cause at least one processor to perform operations, the operations comprising measuring a first blood pressure of a patient to set a first baseline blood pressure, monitoring an estimated blood pressure of the patient for a first period of time, measuring a second blood pressure of the patient in response to a determination that the estimated blood pressure of the patient during the first period of time is greater than a predetermined threshold from the first baseline blood pressure, setting a second baseline blood pressure based on the measuring a second blood pressure of the patient, monitoring an estimated blood pressure of the patient for a second period of time, and measuring a third blood pressure of the patient in response to a determination that the estimated blood pressure of the patient during the second period of time is greater than a predetermined threshold from the second baseline blood pressure.

### BRIEF DESCRIPTION OF THE DRAWINGS

FIG. 1 is a block diagram of an apparatus according to an aspect.
FIG. 2 is a perspective view of an apparatus according to an aspect disposed proximate a body of a patient.
FIG. 3 is a perspective view of an apparatus according to an aspect disposed proximate a body of a patient.
FIGS. 4 and 5 are side views of a monitor of the apparatus of FIG. 2.
FIG. 6 is a block diagram of the monitor of FIGS. 4 and 5.
FIG. 7 is a perspective view of a blood pressure measuring device of the apparatus of FIG. 2
FIG. 8 is a block diagram of the blood pressure measuring device of FIG. 7.
FIG. 9 is a block diagram of a patient status detector of the apparatus of FIG. 2.
FIG. 10 is a block diagram of a controller of the apparatus of FIG. 2.
FIG. 11 is a blood pressure chart.
FIGS. 12A and 12B are blood pressure charts.
FIGS. 13-16 are flow charts of methods according to aspects.

### DETAILED DESCRIPTION

In general, the implementations are directed to medical devices and methods. The term patient or user may hereinafter be used for a person who benefits from the medical device or the methods disclosed in the present disclosure. For example, the patient can be a person whose body contacted by, engaged with, or otherwise interacts with the medical device.

In some implementations, an apparatus or device is configured to trigger blood pressure measurements of a patient at appropriate times and frequencies. In some implementations, the apparatus or device is configured such that blood pressure measurements of the patient are taken at desirable time intervals (and such that the measurements are not taken too frequently). In some implementations, an estimated blood pressure of the patient is used to determine when a blood pressure measurement should be taken. In some such implementations, the baseline used in making such determination is adjusted or changed over time. For example, in some implementations, the baseline used is changed according to a recent blood pressure measurement of the patient.

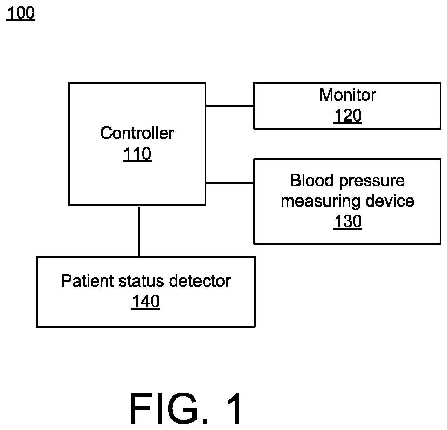

FIG. 1 is a block diagram of an apparatus 100 according to an aspect. The apparatus 100 includes a controller 110, a monitor 120, and a blood pressure measuring device 130. The apparatus 100 also includes a patient status detector 140. The controller 110 is operatively coupled to the monitor 120, to the blood pressure measuring device 130, and to the patient status detector 140. In some aspects, the controller 110 is coupled to such components wirelessly. For example, in some aspects, the controller 110 is operatively coupled to such components via infrared waves, radio frequency, Bluetooth, or a different wireless technology. In other aspects, the controller 110 is operatively coupled to the components via wires or other conduits.

The monitor 120 is configured to monitor an estimated blood pressure of a patient. For example, in some aspects, the monitor 120 is configured to monitor an estimated aortic blood pressure of a patient. In other aspects, the monitor 120 is configured to monitor an estimated different type of blood pressure of a patient. For example, in some aspects, the monitor 120 is configured to monitor an estimated peripheral blood pressure of a patient. In some aspects, as described in more detail below, the monitor 120 is configured to send raw, filtered, or processed photoplethysmogram signal data to the controller 110. The controller 110 may then use such data to determine the estimated blood pressure of the patient.

The monitor 120 is configured to provide signals indicating the estimated blood pressure of the patient to the controller 110. For example, in some aspects, the monitor 120 is configured to provide signals to an input that is operatively coupled to the monitor 120.

In some aspects, the monitor 120 is coupled to a portion of the body of the patient. For example, in some aspects, the monitor 120 is a pulse oximeter and is configured to send photoplethysmogram signals to the controller 110. In such aspects, the monitor 120 may be configured to be disposed adjacent or coupled to a finger of the patient. Accordingly, in some aspects, only a single signal sent from the monitor 120 to the controller 110 is necessary for the monitoring of the estimated blood pressure of the patient. In other aspects, the monitor 120 is configured to be coupled to a different portion of the body of the patient. In other aspects, the monitor 120 is a different type of device, such as a tonometric blood pressure waveform device or an ECG with an impedance cardiogram.

The blood pressure measuring device 130 is configured to measure the blood pressure of a patient. In some aspects, the blood pressure measuring device 130 is a non-invasive blood pressure measuring device, such as a blood pressure cuff. In other aspects, the blood pressure measuring device 130 is a different type of blood pressure measuring device.

In some aspects, the blood pressure measuring device 130 includes a pump or pump system that is operatively coupled to an inflatable member. The pump or pump system is configured to selectively inflate and deflate the inflatable member. For example, in some aspects, the inflatable member includes an inflation portion or a bladder that is configured to receive a fluid such as air. The pump or pump system is configured to deliver the fluid or air to the inflatable portion of the inflatable member to selectively place the inflatable member in different states of inflation (such as a deflated state or an inflated state). In some aspects, the pump or pump system includes a pump or series of pumps and a valve or a series of valves. In some aspects, the pump or pump system is configured to inflate the inflatable member and allow the inflatable member to deflate.

In some aspects, the blood pressure measuring device 130 also includes a sensor that is configured to sense or detect vibrations associated with blood flow within the body of the patient. In other aspects, the blood pressure measuring device 130 includes a sensor that is configured to detect other vibrations or motions.

In some aspects, the blood pressure measuring device 130 is a blood pressure cuff. In such aspects, the blood pressure measuring device 130 is configured to be placed in a variety of different states or inflation configurations. For example, in some aspects, the blood pressure measuring device 130 is configured to be placed in a deflated configuration and an inflated configuration. The pressure within the blood pressure measuring device 130 is greater when the blood pressure measuring device 130 is disposed in the inflated configuration than when the blood pressure measuring device is disposed in the deflated configuration. In some aspects, the blood pressure measuring device 130 is configured to be disposed proximate a portion of a body of a patient and apply varying amounts of pressure against the body of the patient (depending on how inflated the inflatable member is). For example, the blood pressure measuring device 130 may place more pressure on a portion of the body of the patient when the blood pressure measuring device 130 is disposed in an inflated configuration than when the blood pressure measuring device 130 is disposed in the deflated configuration. In some aspects, the blood pressure measuring device 130 includes a chamber or other cavity that is configured to receive air or another fluid to be placed in an inflated configuration.

In some aspects, the blood pressure measuring device 130 is configured to be removably coupled to a portion of a body of a patient. For example, in some aspects, the blood pressure measuring device 130 is configured to be removably coupled to a portion of a body of a patient, such as a portion of an arm or of a leg of the patient.

In some aspects, the controller 110 may be set to cause the blood pressure measuring device 130 to take routine or scheduled blood pressure of the patient at regular intervals. For example, in some aspects, the controller 110 may be configured to cause the blood pressure measuring device 130 to measure the blood pressure of the patient once every 4 hours. In the illustrated aspect, the controller 110 is also configured to cause the blood pressure measuring device 130 to measure the blood pressure of the patient at times that differ from the scheduled times.

For example, in some aspects, the controller 110 is configured to cause the blood pressure measuring device 130 to take a first blood pressure measurement of the patient in response to receiving an estimated blood pressure from the monitor 120 that deviates from a set of rules or standards for taking a blood pressure measurement. For example, in one aspect, the controller 110 is configured to cause the blood pressure measuring device 130 to take a first blood pressure measurement of the patient in response to receiving an estimated blood pressure from the monitor 120 that deviates from a baseline. Additionally, the controller 110 is configured to prevent the blood pressure measuring device 130 from taking a second blood pressure measurement of the patient until a time period has expired since the first blood pressure measurement of the patient. For example, in some aspects, the controller 110 is configured to prevent the blood pressure measuring device 130 from taking a second blood pressure measurement of the patient until a period of at least 10 minutes and less than 30 minutes has past or expired since the first blood pressure measurement of the patient. For example, in some aspects, the controller 110 is configured to prevent the blood pressure measuring device 130 from taking a second blood pressure measurement of the patient until at least 15 minutes has past or expired since the first blood pressure measurement of the patient. In other aspects, a different time amount must lapse (greater than 15 minutes or less than 15 minutes) before the controller 110 allows the taking of a second blood pressure measurement. In other words, even if the controller 110 receives an estimated blood pressure from the monitor 120 that might otherwise cause the monitor 120 to cause a blood pressure measurement, such blood pressure measurement will not occur if sufficient time has not past or expired since a prior blood pressure measurement. Accordingly, the controller 110 is configured to prevent the blood pressure measuring device 130 from taking too frequent blood pressure measurements or from taking measurements that are too close in time to each other.

In some aspects, the controller 110 is configured to cause the blood pressure measuring device 130 to take a first blood pressure measurement of the patient in response to receiving an estimated blood pressure from the monitor 120 that deviates from a first baseline by an amount greater than a threshold or set amount. For example, when a physician or other medical personnel applies the device on the patient, he or she manually triggers a blood pressure measurement. The initial baseline blood pressure is derived from these blood pressure values. This initial blood pressure measurement may be triggered automatically by the controller following the application of the device. Going forward, the baseline may be adjusted each time a blood pressure measurement is taken.

Additionally, the controller 110 is configured to cause the blood pressure measuring device 130 to take a second blood pressure measurement of the patient in response to receiving an estimated blood pressure from the monitor 120 that deviates from a second baseline by an amount greater than a threshold or set amount. In some aspects, the second baseline is different than the first baseline. In some such aspects, the second baseline may be set after the first blood pressure measurement. In other words, the baseline that is used by the monitor 120 to trigger blood pressure measurements may be adjusted, changed, or reset.

In some aspects, an output is operatively coupled to the controller 110 and an input is operatively coupled to the controller 110. The output is configured to provide a signal to cause a blood pressure measurement of a patient to be taken. For example, in some aspects, the output is configured to provide a signal to the blood pressure measuring device 130 to cause the blood pressure measuring device 130 to measure the blood pressure of the patient.

The input is configured to receive a signal when the estimated blood pressure of the patient deviates from a baseline. The controller 110 is configured to cause the blood pressure measuring device 130 to take a blood pressure measurement of the patient in response to the input receiving a signal that an estimated blood pressure of the patient deviates from the baseline.

In some aspects, the controller 110 includes hardware and software. For example, various implementations of the controller 110 described herein can be realized as described in detail below.

The patient status detector 140 is configured to detect a status of the body of the patient. For example, in some aspects, the patient status detector 140 is configured to detect or determine if the body of the patient is upright or if the body of the patient is lying down. In some aspects, the patient status detector 140 is configured to detect or determine if the body of the patient is moving. In some aspects, the patient status detector 140 includes an accelerometer. In other aspects, the patient status detector 140 is or includes a different type of monitor or device.

In some aspects, the patient status detector 140 is a part of or included within the blood pressure measurement device 130. In other aspects, the patient status detector 140 is a separate device or a unit separate from the blood pressure measurement device 130. The patient status detector 140 is configured to be removably coupled to a portion of the body of the patient. In some aspects, the patient status detector 140 is separate from and is configured to be coupled to the body of the patient at a location different than the location where the monitor 120 is coupled to the body of the patient. Similarly, in some aspects, the patient status detector 140 is separate from and is configured to be coupled to the body of the patient at a location different than the location where the blood pressure measuring device 130 is coupled to the body of the patient.

In some aspects, the patient status detector 140 is configured to be removably coupled to a torso, chest, or abdomen area of a patient. In other aspects, the patient status detector 140 is configured to be removably coupled to another portion of the body of the patient.

In use, the apparatus 100 may be placed such that at least a portion of the apparatus 100 is disposed proximate a portion of a body of a patient. For example, the apparatus 100 may be placed such that the monitor 120 is disposed on a hand or a finger of a patient, the blood pressure measuring device is disposed on an arm of the patient, and the patient status detector 140 is disposed on a torso of the patient.

The monitor 120 may then monitor, detect, or otherwise determine an estimated blood pressure of the patient. The monitor 120 sends signals indicative of the estimated blood pressure of the patient to the controller 110. For example, in some aspects, the monitor 120 is configured to send photoplethysmogram signals to the controller 110. The controller 110 may then use those photoplethysmogram signals to determine the estimated blood pressure of the patient. In other aspects, the monitor 120 may send estimated blood pressure measurements to the controller 110. When the controller 110 receives an indication that the estimated blood pressure of the patient has deviated from a baseline (for example if the blood pressure deviates beyond a threshold amount from a baseline), the controller 110 is configured to send a signal or instructions to the blood pressure measuring device 130 to have the blood pressure measuring device 130 activate and take a measurement of the blood pressure of the patient.

In some aspects, the controller 110 is configured to prevent the blood pressure measuring device 130 from taking blood pressure measurements too frequently. For example, in some aspects, even if the blood pressure estimate from the monitor 120 deviates from the baseline, the controller 110 will not send the instructions to have the blood pressure measuring device 130 measure the blood pressure of the patient if sufficient time has not past or expired since a prior blood pressure measurement by the blood pressure measuring device 130. In some aspects, at such times, the controller 110 may send a signal to instruct the blood pressure measuring device 130 to not take a blood pressure measurement of the patient.

In some aspects, the controller is configured to reset or adjust the baseline blood pressure of the patient based on prior blood pressure measurements of the patient. Additionally, in some aspects, the threshold used by the controller 110 to determine if a blood pressure measurement is required is adjustable or selectable. For example, in some aspects, a patient or a physician may determine or select how large the threshold is (or how much the estimated blood pressure needs to deviate from the baseline to cause the controller 110 to instruct that a blood pressure measurement be taken).

In some aspects, the controller 110 is configured to only have the blood pressure of the patient measured if the patient status detector 140 indicates that the patient is not elevated and that the patient is not moving too much to affect a blood pressure measurement.

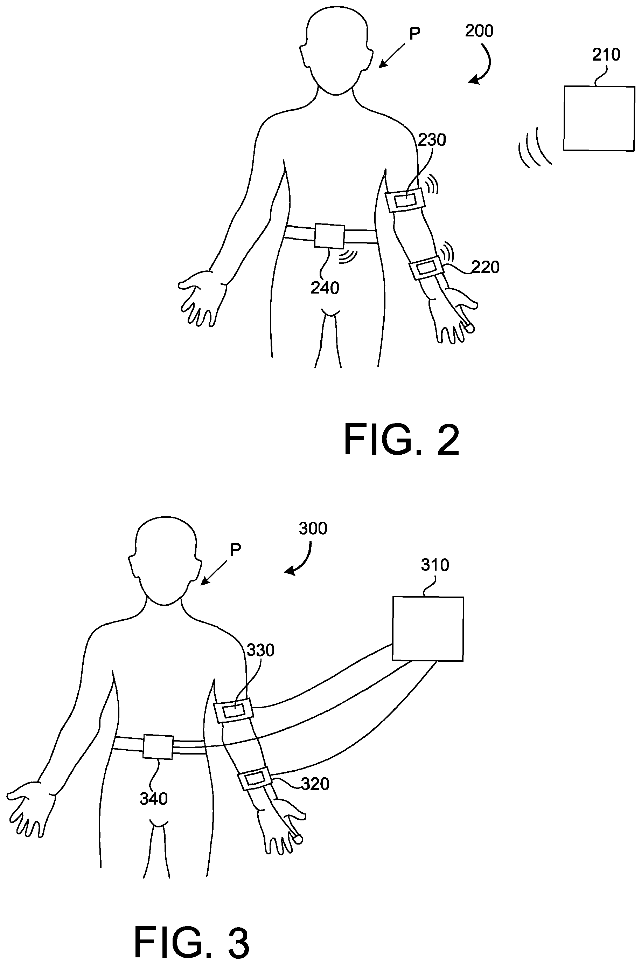

FIG. 2 illustrates an apparatus 200 according to an aspect. The apparatus 200 includes a controller 210, a monitor 220, and a blood pressure measuring device 230. The apparatus 200 also includes a patient status detector 240. In the illustrated aspect, the controller 210 is disposed at a location spaced or separate from the body of the patient P. The monitor 220 is removably coupled to a wrist and finger of the patient P. The blood pressure measuring device 230 is removably coupled to an arm of the patient P. The patient status detector 240 is removably coupled to the torso of the patient P. The controller 210 is operatively coupled to the monitor 220, to the blood pressure measuring device 230, and to the patient status detector 240. In the illustrated aspect, the controller 210 is coupled to the monitor 220, to the blood pressure measuring device 230, and to the patient status detector 240 wirelessly. For example, in some aspects, the controller 210 is operatively coupled to such components via infrared waves, radio frequency, Bluetooth, or a different wireless technology.

In other aspects, the controller is operatively coupled to the components via wires or other conduits. For example, as illustrated in FIG. 3, an apparatus 300 includes a controller 310, a monitor 320, and a blood pressure measuring device 330. The apparatus 300 also includes a patient status detector 340. In the illustrated aspect, the controller 310 is disposed at a location spaced or separate from the body of the patient P. The monitor 320 is removably coupled to a wrist and finger of the patient P. The blood pressure measuring device 330 is removably coupled to an arm of the patient P. The patient status detector 340 is removably coupled to the torso of the patient P. The controller 310 is operatively coupled to the monitor 320, to the blood pressure measuring device 330, and to the patient status detector 340. In the illustrated aspect, the controller 310 is coupled to the monitor 320, to the blood pressure measuring device 330, and to the patient status detector 340 via wires or conduits.

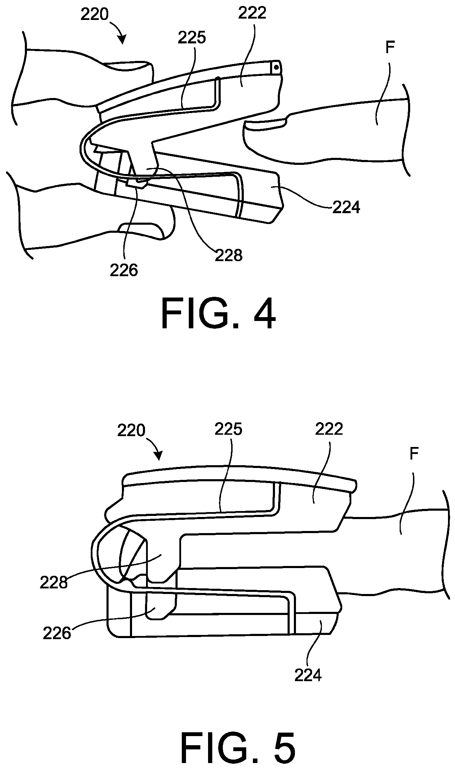

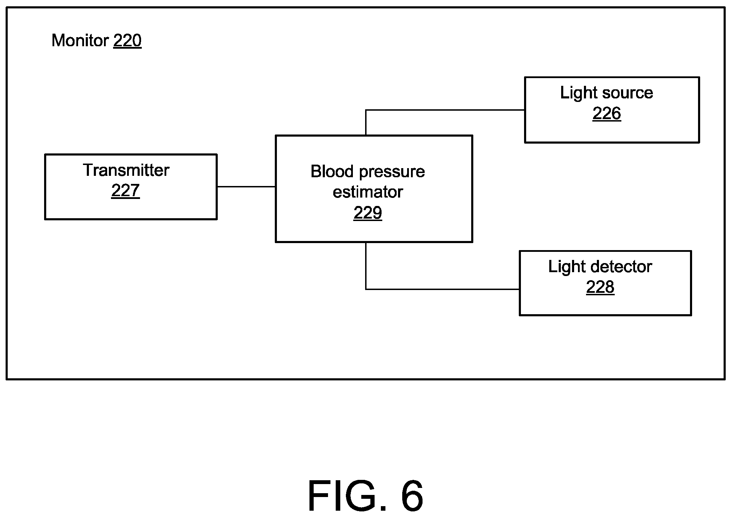

FIGS. 4 and 5 are side views of a monitor 220 of apparatus 200. FIG. 6 is a block diagram of the monitor 220.

The monitor 220 may be configured to monitor an estimated blood pressure of a patient. For example, in some aspects, the monitor 220 is configured to monitor an estimated aortic blood pressure of a patient. In other aspects, the monitor 220 is configured to monitor an estimated different type of blood pressure of a patient. For example, in some aspects, the monitor 220 is configured to monitor an estimated peripheral blood pressure of a patient.

The monitor 220 may be configured to provide signals indicating the estimated blood pressure of the patient to the controller 210 which estimates that blood pressure of the patient. For example, the monitor 220 may be configured to provide signals to the input or the input interface 214 of the control unit 250 that is operatively coupled to the monitor 220.

In the illustrated aspect, the monitor 220 is configured to be disposed adjacent or coupled to a finger F of the patient. As best illustrated in FIGS. 4 and 5, the monitor 220 includes a first portion 222 that is pivotally coupled to a second portion 224. The first portion 222 is configured to pivot with respect to the second portion 224 receive a portion of a finger F of the patient to removably couple the monitor 220 to the finger F of the patient. In the illustrated aspect, the monitor 220 includes a bias member 225. The bias member 225 is configured to bias the first portion 222 and the second portion 224 to a closed position (as illustrated in FIG. 5). Accordingly, the monitor 220 is configured to remain coupled to the finger F of the patient.

In the illustrated aspect, the monitor 220 includes a light source 226 and a light detector 228. In some aspects, the light source 226 and the light detector 228 are configured to monitor or detect physical changes in the patient. In some aspects, the light source 226 and the light detector 228 are configured to detect information indicative of an estimated blood pressure of the patient. Although one light source and one light detector are shown in the illustrated aspect, in some aspects, the monitor includes more than one light source and/or more than one light detector. The monitor 220 is not limited to the light source 226 and light detector 228, and may include additional or alternative sensors for monitoring physiological parameters that may be used to estimate blood pressure.

FIG. 6 is a schematic illustration of the monitor 220. The monitor 220 includes the light source 226 and the light detector 228. The light source 226 and the light detector 228 are operatively coupled to a blood pressure estimator 229. In some aspects, the blood pressure estimator 229 is a module or other device configured to receive information from the light source 226 and the light detector 228 and determine or estimate a blood pressure of the patient. The blood pressure estimator 229 is operatively coupled to a transmitter 227. The transmitter 227 is configured to send information, such as the estimated blood pressure of the patient, to the controller 210. While in the illustrated aspect, the blood pressure estimator 229 is a portion of the monitor 220, in other aspects, the blood pressure estimator 229 is a portion of the controller 210.

In some aspects, the monitor 220 is a pulse oximeter and is configured to send photoplethysmogram signals or other signals indicative of an estimated blood pressure of the patient. In some aspects, the pulse oximeter or the photoplethysmogram signals are configured to estimate a central or aortic blood pressure of the patient. In some aspects, the pulse oximeter detects changes in the aortic blood pressure of the patient by analyzing amplitude and timing differences in the photoplethysmogram signal deflections. In some aspects, the data is received from red or infrared channels of an SpO2 sensor. In some aspects, the photoplethysmogram signals provide for an estimate or determination of the estimated blood pressure of the patient at a rate of about 100 times per second. In some aspects, the photoplethysmogram signal includes signal frequencies of between 0 and 50 Hz. In other aspects, other signal frequencies are used.

In some aspects, the monitor 220 is a pulse oximeter and no additional devices or monitors are needed to estimate the blood pressure of the patient. Accordingly, in some aspects, there is no need to synchronize time-dependent signals of multiple monitors.

The blood pressure measuring device 230 is configured to measure the blood pressure of the patient. In the illustrated aspect, the blood pressure measuring device 230 is a blood pressure cuff.

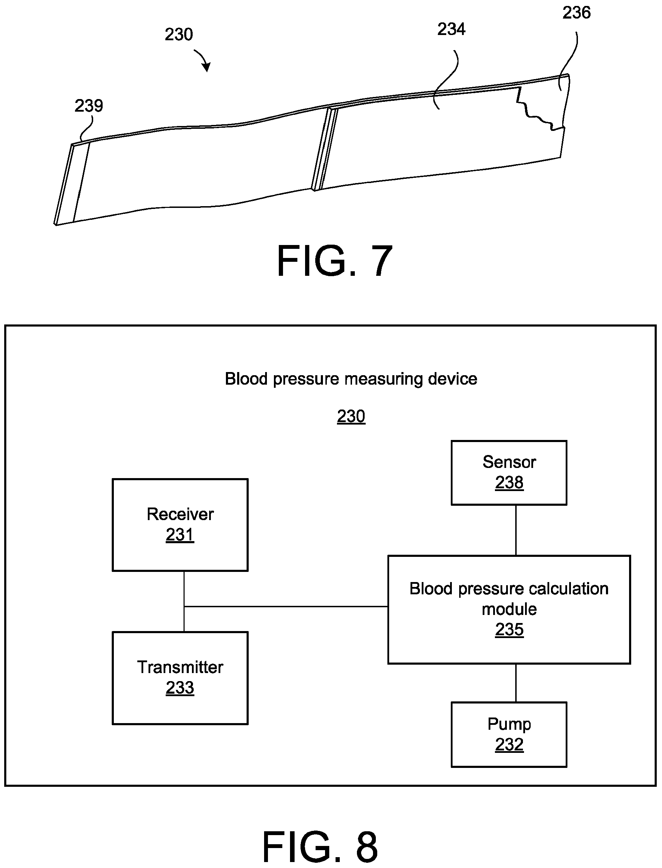

FIG. 7 is a perspective view of the blood pressure measuring device 230 and FIG. 8 schematically illustrates the blood pressure measuring device 230.

The blood pressure measuring device 230 includes a pump or pump system 232 that is operatively coupled to an inflatable member 234. The pump or pump system 232 is configured to selectively inflate and deflate the inflatable member 234. For example, in some aspects, the inflatable member 234 includes an inflation portion or a bladder 236 that is configured to receive a fluid such as air. The pump or pump system 232 is configured to deliver the fluid or air to the inflatable portion 236 of the inflatable member 234 to selectively place the inflatable member 234 in different states of inflation (such as a deflated state or an inflated state). In the illustrated aspect, the blood pressure measuring device 230 also includes a sensor 238 that is configured to sense or detect vibrations associated with blood flow within the body of the patient.

The blood pressure measuring device 230 is configured to be placed in a variety of different states or inflation configurations. For example, in some aspects, the blood pressure measuring device 230 is configured to be placed in a deflated configuration and an inflated configuration. The pressure within the blood pressure measuring device 230 is greater when the blood pressure measuring device 230 is disposed in the inflated configuration than when the blood pressure measuring device is disposed in the deflated configuration. In some aspects, the blood pressure measuring device 230 is configured to be disposed proximate a portion of a body of a patient and apply varying amounts of pressure against the body of the patient (depending on how inflated the inflatable member is). For example, the blood pressure measuring device 230 may place more pressure on a portion of the body of the patient when the blood pressure measuring device 230 is disposed in an inflated configuration than when the blood pressure measuring device 230 is disposed in the deflated configuration.

In the illustrated aspect, the blood pressure measuring device 230 includes a coupling portion 239. In the illustrated aspect, the coupling portion 239 may be used to removably couple the blood pressure measuring device 230 to an arm of a user. Specifically, the blood pressure measuring device 230 may be wrapped around an arm of the patient and the coupling portion 239 may removably couple to another portion of the blood pressure measuring device 230 to form a loop or cuff around the arm of the patient.

In some aspects, the coupling portion 239 includes hook material or loop material or another type of material that is configured to be coupled to, such as removably coupled to, another portion of the blood pressure measuring device 230.

In the illustrated aspect, the blood pressure measuring device 230 includes a blood pressure calculation module 235. While the blood pressure calculation module 235 is shown as part of the blood pressure measuring device 230 in the illustrated aspect, in other aspects, the blood pressure calculation module 235 may reside in a different portion or unit of the device. For example, in some aspects, the blood pressure calculation module 235 is disposed within or a portion of the controller 110. The blood pressure calculation module 235 is operatively coupled to the sensor 238 and to the pump or pump system 232. The blood pressure calculation module 235 is configured to receive information from the sensor 238 and the pump or pump system 232 and calculate or determine the blood pressure of the patient. In some aspects, the blood pressure calculation module 235 is configured to calculate or determine a systolic blood pressure and a diastolic blood pressure of the patient.

The blood pressure measuring device 230 includes a receiver 231 and a transmitter 233. The receiver 231 is operatively coupled to the controller 210. In some aspects, the receiver 231 is configured to receive instructions from the controller 210. For example, in some aspects, the receiver 231 is configured to receive instructions to measure the blood pressure of the patient. The transmitter 233 is operatively coupled to the controller 210. In some aspects, the transmitter 233 is configured to provide information, such as the blood pressure of the patient, to the controller 210.

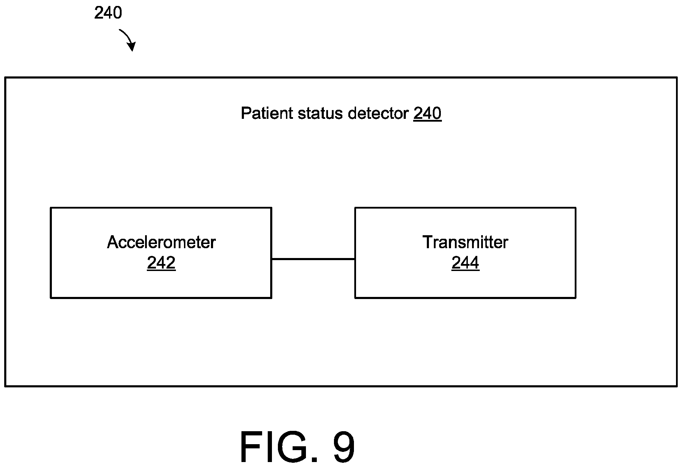

FIG. 9 is a block diagram of an example of a patient status detector 240. The patient status detector 240 is configured to detect a status of the body of the patient. For example, the patient status detector 240 is configured to detect or determine if the body of the patient is upright or if the body of the patient is lying down. Additionally, the patient status detector 240 is configured to detect or determine if the body of the patient is moving.

The patient status detector 240 is configured to be removably coupled to a portion of the body of the patient. The patient status detector 240 is separate from and is configured to be coupled to the body of the patient at a location different than the location where the monitor 220 is coupled to the body of the patient. Similarly, the patient status detector 240 is separate from and is configured to be coupled to the body of the patient at a location different than the location where the blood pressure measuring device 230 is coupled to the body of the patient.

In the illustrated aspect, the patient status detector 240 is configured to be removably coupled to a torso of the patient. In other aspects, the patient status detector 240 is configured to be removably coupled to another portion of the body of the patient.

In the illustrated aspect, the patient status detector 240 includes an accelerometer 242 and a transmitter 244. The accelerometer 242 is configured to detect whether the patient is upright or lying down. The accelerometer 242 is also configured to determine if a patient is moving. The transmitter 244 is operatively coupled to the controller 210. The transmitter 244 is configured to provide information or data such as whether the patient is upright or lying down and whether the patient is moving to the controller 210.

The controller 210 may be configured to cause the blood pressure measuring device 230 to take a blood pressure measurement of the patient in response to receiving an estimated blood pressure from the monitor 220 that deviates from a baseline. For example, in some aspects, the monitor 220 is configured to cause the blood pressure measuring device 230 to take a blood pressure measurement in response to receiving an estimated blood pressure of the patient from the monitor 220 that deviates from the baseline blood pressure by an amount greater than a threshold amount. In some aspects, the threshold amount may be an absolute amount, such as plus or minus a specified amount of pressure. In other aspects, the threshold may be a percentage of the blood pressure of the patient. In other words, the threshold may be plus or minus 5% or plus or minus 10% of the blood pressure of the patient.

In some aspects, the controller 210 is configured to cause the blood pressure measuring device 230 to take a blood pressure measurement of the patient in response to receiving an indication from the monitor 220 that the estimated blood pressure of the patient deviates from the baseline for a sustained period of time. For example, in some aspects, the estimated blood pressure must deviate from the baseline by an amount exceeding the threshold amount for a few seconds or a few minutes before the controller 210 causes the blood pressure measuring device 230 to take a blood pressure measurement. Accordingly, the controller 210 may not cause the blood pressure of the patient to be measured due to a single or a couple of inaccurate estimates of the blood pressure of the patient.

Additionally, the controller 210 may be configured to prevent the blood pressure measuring device 230 from taking a second or subsequent blood pressure measurement of the patient until a time period has expired since the first or previous blood pressure measurement of the patient. For example, in some aspects, the controller 210 is configured to prevent the blood pressure measuring device 230 from taking a second blood pressure measurement of the patient until a period of at least 10 minutes and less than 30 minutes has past or expired since the first blood pressure measurement of the patient. For example, in some aspects, the controller 210 is configured to prevent the blood pressure measuring device 230 from taking a second blood pressure measurement of the patient until at least 15 minutes has past or expired since the first blood pressure measurement of the patient. In other aspects, a different time amount must lapse (greater than 15 minutes or less than 15 minutes) before the controller 210 allows the taking of a second or subsequent blood pressure measurement. In some aspects, 10 minutes must elapse or expire before the controller 210 allows a second or subsequent blood pressure measurement to be taken. In other aspects, 20 minutes or 25 minutes must elapse or expire before the controller 210 allows a second or subsequent blood pressure measurement. In other words, even if the controller 210 receives an estimated blood pressure from the monitor 220 that might otherwise cause the monitor 220 to trigger a blood pressure measurement, such blood pressure measurement will not occur if sufficient time has not past or expired since a prior blood pressure measurement. Accordingly, the controller 210 is configured to prevent the blood pressure measuring device 230 from taking too frequent blood pressure measurements or from taking measurements that are too close in time to each other.

In the illustrated aspect, the controller 210 is configured to cause the blood pressure measuring device 230 to take a first blood pressure measurement of the patient in response to receiving an estimated blood pressure from the monitor 220 that deviates from a first baseline by an amount greater than a threshold or set amount. Additionally, the controller 210 is configured to cause the blood pressure measuring device 230 to take a second or subsequent blood pressure measurement of the patient in response to receiving an estimated blood pressure from the monitor 220 that deviates from a second baseline by an amount greater than a threshold or set amount.

In some aspects, the controller 210 is configured to increase the threshold if a patient is moving or involved in another activity at the time of the estimated blood pressure. For example, if the patient is moving from a supine to an upright position (or vice versa), the threshold may be adjusted to accommodate the expected blood pressure change. In such aspects, the controller 210 is configured to receive patient activity information from the patent status detector 240.

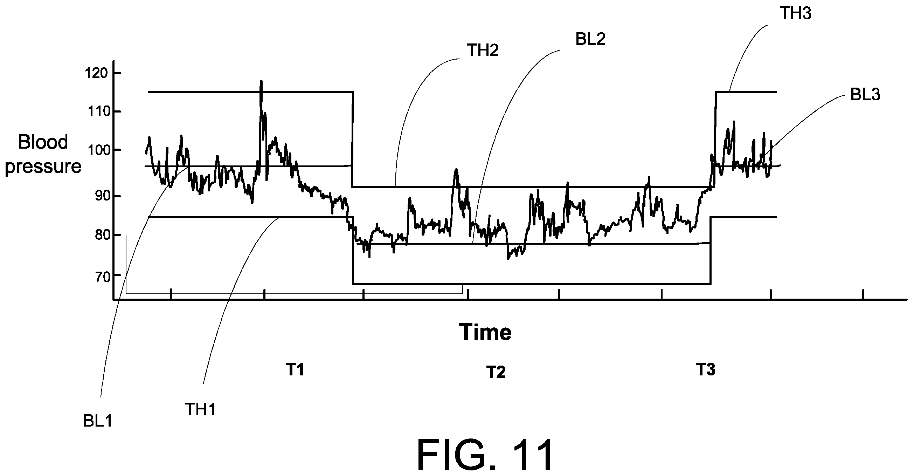

FIG. 11 is a graph illustrating the estimated blood pressure of the patient over a period of time. In one example, this estimated blood pressure is the estimated blood pressure measured by the monitor 220 and provided to the controller 210. The estimated blood pressure of the patient may be a systolic blood pressure, a diastolic blood pressure, or a combination of the two. As shown in FIG. 11, at time T1, a baseline BL1 and a threshold TH1 is set. The estimated blood pressure of the patient drops below the threshold between time T1 and time T2. In some aspects, the controller 210 would then cause the blood pressure measuring device 230 to measure the blood pressure of the patient. The new blood pressure of the patient would then be used to set a new baseline BL2 at time T2. Additionally, a new threshold TH2 would be set. At a point in time between time T2 and time T3, the estimated blood pressure of the patient rises and the controller 210 would cause the blood pressure measuring device 230 to measure the blood pressure of the patient. The new blood pressure of the patient would then be used to set a new baseline BL3 at time T3. Additionally, a new threshold TH3 would be set.

Accordingly, in the illustrated aspect, the second baseline BL2 is different than the first baseline BL1. The second baseline is set after a blood pressure measurement of the patient. In other words, the baseline that is used by the monitor 220 to trigger blood pressure measurements may is adjusted, changed, or reset.

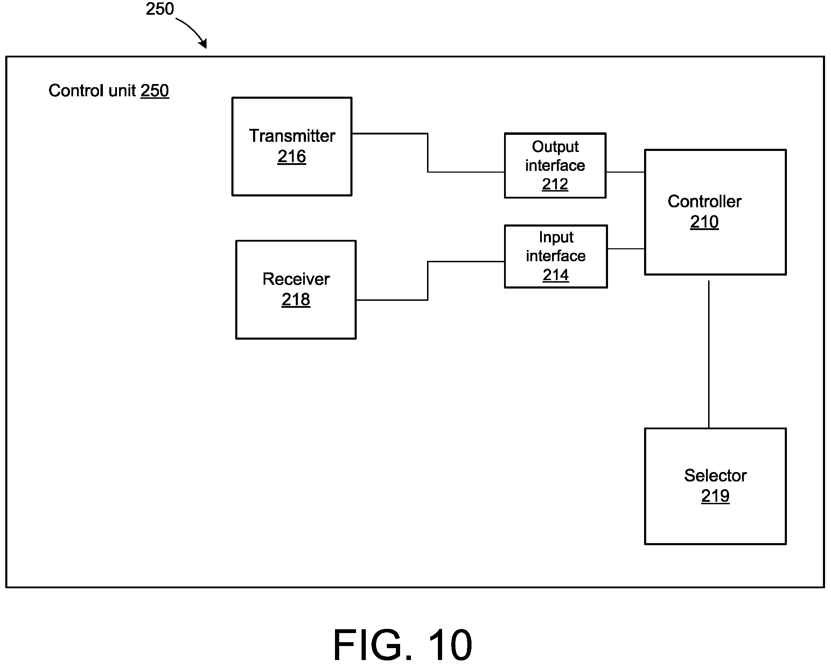

As best illustrated in FIG. 10, the controller 210 is disposed within the control unit 250. In other aspects, the controller 210 is disposed in a different housing or unit or is not part of another unit or housing.

An output or output interface 212 is operatively coupled to the controller 210 and an input or input interface 214 is operatively coupled to the controller 210. The output 212 is configured to provide a signal to cause a blood pressure measurement of a patient be taken. For example, in some aspects, the output is configured to provide a signal to the blood pressure measuring device 230 to cause the blood pressure measuring device 230 to measure the blood pressure of the patient. In the illustrated aspect, the control unit 250 includes a transmitter 216. The transmitter 216 is operatively coupled to the output 212 and to the blood pressure measuring device 230. Accordingly, the controller 210 and the control unit 250 can send wireless signals to the blood pressure measuring device 230.

The input 214 is configured to receive a signal when the estimated blood pressure of the patient deviates from a baseline. The controller 210 is configured to cause the blood pressure measuring device 230 to take a blood pressure measurement of the patient in response to the input 214 receiving a signal that an estimated blood pressure of the patient deviates from the baseline. In the illustrated aspect, a receiver 218 is operatively coupled to the input 214, to the monitor 220, and to the blood pressure measuring device 230. Accordingly, the controller 210 and the control unit 250 can receive wireless signals from the monitor 220 and from the blood pressure measuring device 230. In some aspects, the controller 210 is configured to receive data from the monitor 220 and is configured to determine or estimate the blood pressure of the patient. For example, the controller 210 may determine or estimate the blood pressure of the patient based on the date received from the monitor 220.

**In** the illustrated aspect, the control unit 250 includes a selector or a selection module 219. The selector 219 allows a user or a physician to change various settings of the device 200. For example, in some aspects, the selector 219 allows the user or the physician to select how sensitive to changes in the estimated blood pressure of the user the device 200 is. For example, in some aspects, the selector 219 allows the user or the physician to select how large the threshold is. In such aspects, the user or the physician may select a large threshold (controller 210 may not cause as many blood pressure measurements to be taken) or a small threshold (controller 210 may cause more blood pressure measurements to be taken).

In some aspects, the controller 210 includes hardware and software. For example, various implementations of the controller 210 described herein can be realized as described in detail below.

In use, the apparatus 200 may be placed such that at least a portion of the apparatus 200 is disposed proximate a portion of a body of a patient. For example, the apparatus 200 may be placed such that the monitor 220 is disposed on a hand or a finger of a patient, the blood pressure measuring device 230 is disposed on an arm of the patient, and the patient status detector 240 is disposed on a torso of the patient.

The monitor 220 may then monitor, detect, or otherwise determine an estimated blood pressure of the patient. The monitor 220 sends signals indicative of the estimated blood pressure of the patient to the controller 210. When the controller 210 receives an indication that the estimated blood pressure of the patient has deviated from a baseline (for example if the blood pressure deviates beyond a threshold amount from a baseline), the controller 210 is configured to send a signal or instructions to the blood pressure measuring device 230 to have the blood pressure measuring device 230 activate and take a measurement of the blood pressure of the patient.

In some aspects, the controller 210 is configured to prevent the blood pressure measuring device 230 from taking blood pressure measurements too frequently. For example, in some aspects, even if the blood pressure estimate from the monitor 220 deviates from the baseline, the controller 210 will not send the instructions to have the blood pressure measuring device 230 measure the blood pressure of the patient if sufficient time has not past or expired since a prior blood pressure measurement by the blood pressure measuring device 230. In some aspects, at such times, the controller 210 may send a signal to instruct the blood pressure measuring device 230 to not take a blood pressure measurement of the patient.

As described above, the controller 210 is configured to reset or adjust the baseline blood pressure of the patient based on prior blood pressure measurements of the patient. Additionally, the threshold used by the controller 210 to determine if a blood pressure measurement is required is adjustable or selectable. Specifically, a patient or a physician may determine or select how large the threshold is (or how much the estimated blood pressure needs to deviate from the baseline to cause the controller 210 to instruct that a blood pressure measurement be taken).

In the illustrated aspect, the controller 210 is configured to only have the blood pressure of the patient measured if the patient status detector 240 indicates that the patient is not elevated and that the patient is not moving too much to affect a blood pressure measurement.

According to an aspect of the disclosure, the controller 210 is configured to set or adjust the NIBP measurement triggering threshold based on a relationship between the baseline blood pressure and an alarm limit. Based on the baseline blood pressure being in a normal range (e.g. far from the alarm limits), the threshold may be set to a high value. Based on the baseline blood pressure being in a high or low range (e.g. close to an alarm limit), the threshold may be set to a low value. As such, the sensitivity for triggering a blood pressure can be automatically adjusted based on a state of the patient which may results in disturbing the patient less often for blood pressure measurements.

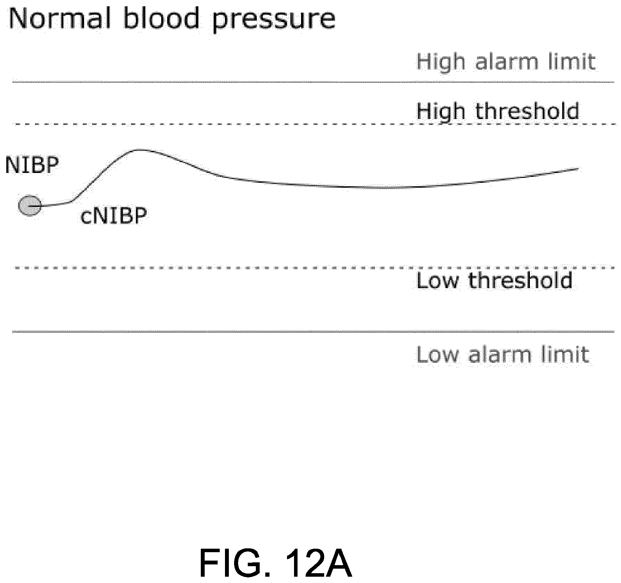

FIG. 12A shows blood pressure data in which the NIPB measurement is in a normal range. Since the blood pressure in in the normal range, and thus not close to the alarm limits, the threshold for trigger another NIBP measurement are a large value. As shown in FIG. 12A, the estimated blood pressure extending out from the NIBP measurement (baseline) has deviations. However, since the threshold is set to a large value, the deviations do not reach the threshold and thus do not trigger another NIBP measurement. Since larger deviations are less problematic when the patient's blood pressure is in a normal range, the larger threshold allows for these large, not problematic deviations without triggering an NIBP measurement.

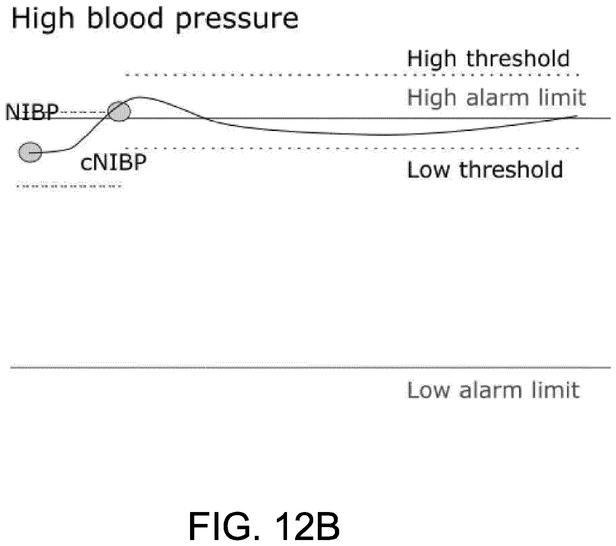

FIG. 12B shows blood pressure data in which the NIPB measurement is in a high range. Since the blood pressure in in the high range, and thus close to the alarm limits, the threshold for trigger another NIBP measurement are small. As shown in FIG. 12B, the estimated blood pressure shows the same deviation from baseline as in FIG. 12A. However, since the threshold is set to a small value in FIG. 12B, the deviation from baseline triggers another NIBP measurement.

As also shown in FIG. 12B, once the threshold is exceeded, another NIBP measurement is taken to obtain a new baseline. New threshold a set based on the new baseline. As such, the new threshold may be smaller than before the baseline measurement because the new baseline is closer to the alarm limit than the initial baseline.

For example, considering a case in which alarm limits are set at 180 mmHg and 60 mmHg and a patient's baseline blood pressure is 120mmHg, the patient's baseline is 60 mmHg from the closest alarm limit. According to an embodiment, the triggering threshold value may be set to 50% of the difference between the baseline and the closest alarm limit. Accordingly, in the above scenario, the threshold value may be set to 30 mmHg resulting in the high threshold being at 150 mmHg and the low threshold being at 90 mmHg.

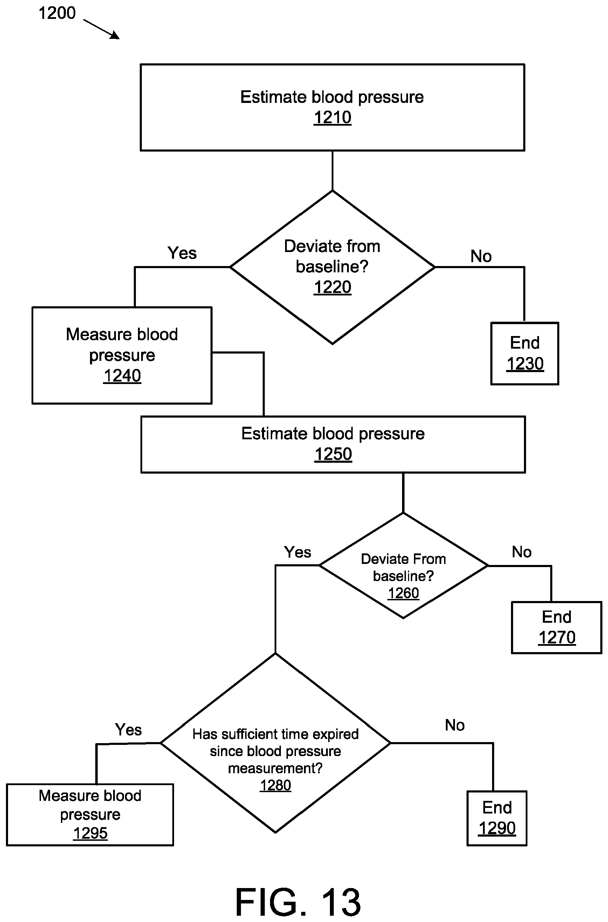

FIG. 13 is a flow chart of a method 1200 according to an aspect. At 1210, the blood pressure of a patient is estimated. In some aspects, the estimated blood pressure of the patient is detected by a monitor and is provided or sent to a controller. At 1220, it is determined whether the estimated the blood pressure of the patient deviates from a baseline. In some aspects, it is determined whether the estimated blood pressure deviates from a baseline by an amount that exceeds a threshold. At 1230, if the estimated the blood pressure does not deviate from the baseline (or does not deviate by an amount that exceeds the threshold), the method is ended.

At 1240, if the estimated blood pressure deviates from the baseline, the blood pressure of the patient is measured. In some aspects, the controller provides instructions or a signal to a blood pressure measuring device to measure the blood pressure of the patient. In other aspects, the user or the patient or a member of the medical staff is notified to take a blood pressure measurement. For example, in some aspects, the blood pressure measuring device may not always be coupled or disposed adjacent the body of the patient. In such aspects, the patient or a member of the medical staff may be notified that a blood pressure measurement should be taken and the patient or the member of the medical staff can then put the blood pressure measuring device on their body (such as put a blood pressure cuff on their arm).aspect

At 1250, the blood pressure of the patient is estimated. In some aspects, the estimated blood pressure of the patient is detected by a monitor and is provided or sent to a controller. At 1260, it is determined whether the estimated the blood pressure of the patient deviates from a baseline. In some aspects, it is determined whether the estimated blood pressure deviates from a baseline by an amount that exceeds a threshold. In some aspects, the baseline that is used at 1260 is different than the baseline that is used at 1220. At 1270, if the estimated the blood pressure does not deviate from the baseline (or does not deviate by an amount that exceeds the threshold), the method is ended.

At 1280, it is determined if sufficient time has expired since the blood pressure measurement that was taken at 1240. In some examples, it is determined if 15 minutes has past or expired since the blood pressure measurement that was taken at 1240. If other examples, it is determined if more than 20 minutes or more has past or expired since the blood pressure measurement that was taken at 1240. At 1290, if a sufficient amount of time has not past or expired, the method is ended. At 1295, if a sufficient amount of time has past or expired since the blood pressure of the patient is measured. In some aspects, a blood pressure cuff is used to measure the blood pressure of the patient.

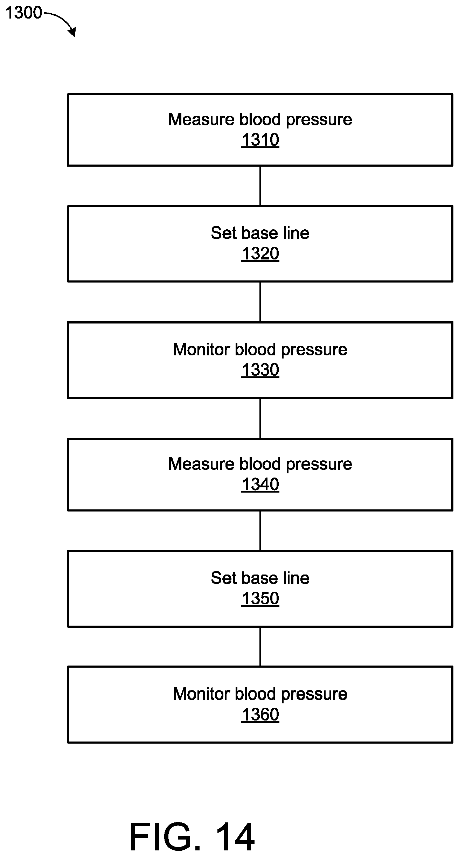

FIG. 14 is a method 1300 according to an aspect. At 1310, a blood pressure of a patient is measured. In some aspects, a blood pressure of the patient is measured using a blood pressure cuff. At 1320, a baseline blood pressure is set based on the measured blood pressure of the patient.

At 1330, a blood pressure of a patient is monitored. In some aspects, a pulse oximeter may be used for monitoring the blood pressure. In some aspects, the monitored blood pressure is an estimated blood pressure and is compared against the baseline blood pressure. At 1340, if the estimated blood pressure deviates from the baseline blood pressure, the blood pressure is again measured. In some aspects, the blood pressure of the patient is measured using a blood pressure cuff. At 1350, a second, new baseline blood pressure is set based on the measured blood pressure (for example, the blood pressured measured at 1340). At 1360, the blood pressure is monitored using the monitor and can be compared against the second, new baseline.

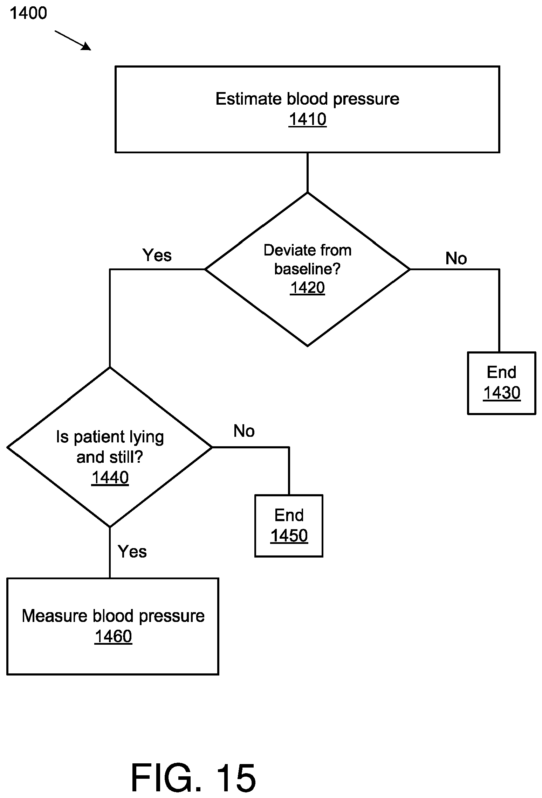

FIG. 15 is a flow chart of a method 1400 according to an aspect. At 1410, the blood pressure of a patient is estimated. In some aspects, the estimated blood pressure of the patient is detected by a monitor and is provided or sent to a controller. At 1420, it is determined whether the estimated the blood pressure of the patient deviates from a baseline. In some aspects, it is determined whether the estimated blood pressure deviates from a baseline by an amount that exceeds a threshold. At 1430, if the estimated the blood pressure does not deviate from the baseline (or does not deviate by an amount that exceeds the threshold), the method is ended.

At 1440, if the estimated blood pressure deviates from the baseline, it is determined if the patient is lying down, or is still, or both. For example, in some aspects, a patient monitor is configured to determine if the patient is lying down and is still sufficient to accurately measure a blood pressure of the patient. In some aspects, the patient monitor includes an accelerometer.

At 1460, if the patient is lying down and is still, the blood pressure of the patient is measured. In some aspects, the controller provides instructions or a signal to a blood pressure measuring device to measure the blood pressure of the patient. In other aspects, the user or the patient or a member of the medical staff is notified to take a blood pressure measurement. For example, in some aspects, the blood pressure measuring device may not always be coupled or disposed adjacent the body of the patient. In such aspects, the patient or the member of the medical staff may be notified that a blood pressure measurement should be taken and the patient or the member of the medical staff can then put the blood pressure measuring device on their body (such as put a blood pressure cuff on their arm).

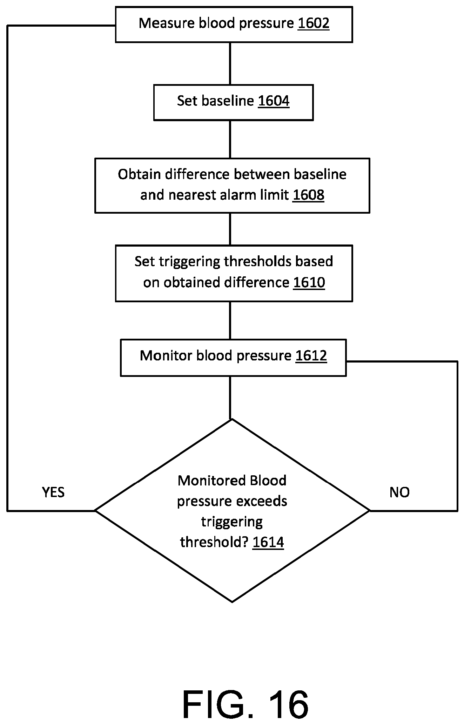

FIG. 16 is a flowchart of method 1600 according to an aspect. At 1602, the blood pressure is measured. For example, the blood pressure may be measured using a NIBP measurement device such as a monitor that uses an inflatable cuff.

At 1604, a baseline blood pressure is set. The baseline blood pressure may be set based on the output of the NIBP measurement device. For example, if the NIBP measurement device outputs 120 mmHg systolic blood pressure, the baseline blood pressure may be set to 120 mmHg. According to some embodiment, the baseline may be set based on an average of multiple measurements. The baseline may be set based on systolic or diastolic blood pressure.

At 1608, a difference between the baseline and a nearest alarm limit is obtained. For example, if the baseline is 140 mmHg and the alarm limits are at 180 mmHg and 60 mmHg, the difference between the baseline and a nearest alarm limit is 40 mmHg.

At 1610, triggering thresholds are obtained based on the determined difference. For example, is the difference is 60 mmHg, a threshold value may be set to 50% of the difference which is 30 mmHg. The triggering thresholds may then be set by adding and subtracting 30 mmHg from the baseline of 140 mmHg to obtain thresholds at 170 mmHg and 110 mmHg.

At 1612, the blood pressure is monitored. For example, the blood pressure may be monitored by an optical device such as a pulsed oximeter. Using data produced by the optical device, a change in blood pressure from the baseline may be obtained to estimate the blood pressure.

At 1614, the monitored blood pressure is compared to the triggering thresholds. Based on the monitored blood pressure not exceeding either of the triggering thresholds (e.g. higher than 170 mmHg or lower than 110 mmHg, the method may revert back to operation 1612. Based on the monitored blood pressure exceeding one of the triggering thresholds, the method may revert back to operation 1602.

Various implementations of the systems, such as the controller and other system, modules, and other units described herein, and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device. Various implementations of the systems and techniques described here can be realized as and/or generally be referred to herein as a circuit, a module, a block, or a system that can combine software and hardware aspects. For example, a module may include the functions/acts/computer program instructions executing on a processor (e.g., a processor formed on a silicon substrate, a GaAs substrate, and the like) or some other programmable data processing apparatus.

Some of the above example aspects are described as processes or methods depicted as flowcharts. Although the flowcharts describe the operations as sequential processes, many of the operations may be performed in parallel, concurrently or simultaneously. In addition, the order of operations may be re-arranged. The processes may be terminated when their operations are completed, but may also have additional steps not included in the figure. The processes may correspond to methods, functions, procedures, subroutines, subprograms, etc.

Methods discussed above, some of which are illustrated by the flow charts, may be implemented by hardware, software, firmware, middleware, microcode, hardware description languages, or any combination thereof. When implemented in software, firmware, middleware or microcode, the program code or code segments to perform the necessary tasks may be stored in a machine or computer readable medium such as a storage medium. A processor(s) may perform the necessary tasks.

Specific structural and functional details disclosed herein are merely representative for purposes of describing example aspects. Example aspects, however, be embodied in many alternate forms and should not be construed as limited to only the aspects set forth herein.

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of example aspects. As used herein, the term and/or includes any and all combinations of one or more of the associated listed items.

The terminology used herein is for the purpose of describing particular aspects only and is not intended to be limiting of example aspects. As used herein, the singular forms "a", "an", and "the" are intended to include the plural forms as well, unless the context clearly indicates otherwise. It will be further understood that the terms comprises, comprising, includes and/or including, when used herein, specify the presence of stated features, integers, steps, operations, elements and/or components, but do not preclude the presence or addition of one or more other features, integers, steps, operations, elements, components and/or groups thereof.

It should also be noted that in some alternative implementations, the functions/acts noted may occur out of the order noted in the figures. For example, two figures shown in succession may in fact be executed concurrently or may sometimes be executed in the reverse order, depending upon the functionality/acts involved.

Unless otherwise defined, all terms (including technical and scientific terms) used herein have the same meaning as commonly understood by one of ordinary skill in the art to which example aspects belong. It will be further understood that terms, e.g., those defined in commonly used dictionaries, should be interpreted as having a meaning that is consistent with their meaning in the context of the relevant art and will not be interpreted in an idealized or overly formal sense unless expressly so defined herein.

Portions of the above example aspects and corresponding detailed description are presented in terms of software, or algorithms and symbolic representations of operation on data bits within a computer memory. These descriptions and representations are the ones by which those of ordinary skill in the art effectively convey the substance of their work to others of ordinary skill in the art. An algorithm, as the term is used here, and as it is used generally, is conceived to be a self-consistent sequence of steps leading to a desired result. The steps are those requiring physical manipulations of physical quantities. Usually, though not necessarily, these quantities take the form of optical, electrical, or magnetic signals capable of being stored, transferred, combined, compared, and otherwise manipulated. It has proven convenient at times, principally for reasons of common usage, to refer to these signals as bits, values, elements, symbols, characters, terms, numbers, or the like.

In the above illustrative aspects, reference to acts and symbolic representations of operations (e.g., in the form of flowcharts) that may be implemented as program modules or functional processes include routines, programs, objects, components, data structures, etc., that perform particular tasks or implement particular abstract data types and may be described and/or implemented using existing hardware at existing structural elements. Such existing hardware may include one or more Central Processing Units (CPUs), digital signal processors (DSPs), application-specific-integrated-circuits, field programmable gate arrays (FPGAs) computers or the like.

It should be borne in mind, however, that all of these and similar terms are to be associated with the appropriate physical quantities and are merely convenient labels applied to these quantities. Unless specifically stated otherwise, or as is apparent from the discussion, terms such as processing or computing or calculating or determining of displaying or the like, refer to the action and processes of a computer system, or similar electronic computing device, that manipulates and transforms data represented as physical, electronic quantities within the computer system's registers and memories into other data similarly represented as physical quantities within the computer system memories or registers or other such information storage, transmission or display devices.

Note also that the software implemented aspects of the example aspects are typically encoded on some form of non-transitory program storage medium or implemented over some type of transmission medium. The program storage medium may be magnetic (e.g., a floppy disk or a hard drive) or optical (e.g., a compact disk read only memory, or CD ROM), and may be read only or random access. Similarly, the transmission medium may be twisted wire pairs, coaxial cable, optical fiber, or some other suitable transmission medium known to the art. The example aspects not limited by these aspects of any given implementation.

Detailed implementations are disclosed herein. However, it is understood that the disclosed implementations are merely examples, which may be embodied in various forms. Therefore, specific structural and functional details disclosed herein are not to be interpreted as limiting, but merely as a basis for the claims and as a representative basis for teaching one skilled in the art to employ the implementations in virtually any appropriately detailed structure. Further, the terms and phrases used herein are not intended to be limiting, but to provide an understandable description of the present disclosure.

It should also be noted that whilst the accompanying claims set out particular combinations of features described herein, the scope of the present disclosure is not limited to the particular combinations hereafter claimed, but instead extends to encompass any combination of features or aspects herein disclosed irrespective of whether or not that particular combination has been specifically enumerated in the accompanying claims at this time. Additionally, while certain features of the described implementations have been illustrated as described herein, many modifications, substitutions, changes and equivalents will now occur to those skilled in the art. The current invention is defined by the claims.

## Claims

1. An apparatus (100, 200, 300), comprising:
a monitor (120, 220, 320) configured to estimate a blood pressure of a patient;
a blood pressure measuring device (130, 230, 330) configured to measure a blood pressure of the patient;
a controller (110, 210, 310) operatively coupled to the monitor (120, 220, 320) and to the blood pressure measuring device (130, 230, 330), the controller (110, 210, 310) configured to cause the blood pressure measuring device (130, 230, 330) to take a first blood pressure measurement of the patient in response to a first estimated blood pressure deviating from a first baseline by a first threshold amount, the controller (110, 210, 310) being configured to cause the blood pressure measuring device (130, 230, 330) to take a second blood pressure measurement (130, 230, 330) of the patient in response to a second estimated blood pressure deviating from a second baseline different than the first baseline by a second threshold amount,
**characterised in that**
the controller (110, 210, 310) is configured to obtain the first threshold amount based on a difference between the first baseline and an alarm limit, and to obtain the second threshold amount based on a difference between the second baseline and an alarm limit; and
a patient status detector (140, 240, 340) operatively coupled to the controller, wherein the patient status detector (140, 240, 340) comprises an accelerometer configured to determine whether the patient is lying down and whether the patient is not moving;
wherein the controller is configured to cause the blood pressure measuring device to take a blood pressure measurement of the patient only if the patient status detector indicates the patient is not elevated and the patient is not moving too much to affect the measurement.

2. The apparatus of claim 1, wherein the controller (110, 210, 310) is configured to prevent the blood pressure measuring device (130, 230, 330) from taking the second blood pressure measurement until a time period has expired since the first blood pressure measurement of the patient.

3. The apparatus of any of claims 1-2, wherein the first baseline is set based on a measured blood pressure of the patient.

4. The apparatus of any of claims 1-3, wherein the monitor (120, 220, 320) is configured to estimate an aortic blood pressure of the patient.

5. The apparatus of any of claims 1-3, wherein the monitor (120, 220, 320) is a pulse oximeter

6. The apparatus of any of claims 1-5, wherein the blood pressure measuring device (130, 230, 330) includes an inflatable cuff.

7. The apparatus of any of claims 1-6, wherein the controller (110, 210, 310) is configured to cause the blood pressure measuring device to take the first blood pressure measurement of the patient in response to the first estimated blood pressure that deviates from the first baseline by an amount greater than a threshold amount.

8. The apparatus of any of claims 1-7, further comprising:
an input interface (214) configured to receive a signal indicative of a blood pressure estimate of a patient;
an output interface (212) configured to provide a signal to take a first blood pressure measurement of the patient in response receiving a signal indicating that an estimated blood pressure of the patient deviates from a baseline; and
a controller (110, 210, 310) coupled to the input interface (214) and to the output interface (212) and configured to prevent a second blood pressure measurement of the patient until a time period has expired since the first blood pressure measurement of the patient.

9. The apparatus of claim 8, wherein the input interface (214) is configured to be operatively coupled to a pulse oximeter.

10. The apparatus of any of claims 8-9, wherein the output interface (212) is configured to be operatively coupled to a non-invasive blood pressure measuring device (130, 230, 330).

11. The apparatus of any of claims 8-9, wherein the output interface (212) is configured to be operatively coupled to a blood pressure cuff.

12. The apparatus of claim 8, further comprising:
a patient status detector (140, 240, 340) operatively coupled to the controller.

## Patentansprüche

1. Einrichtung (100, 200, 300), umfassend:
einen Monitor (120, 220, 320), der dazu ausgelegt ist, einen Blutdruck eines Patienten zu schätzen;
eine Blutdruckmessvorrichtung (130, 230, 330), die dazu ausgelegt ist, einen Blutdruck des Patienten zu messen;
eine Steuerung (110, 210, 310), die operativ mit dem Monitor (120, 220, 320) und der Blutdruckmessvorrichtung (130, 230, 330) gekoppelt ist, wobei die Steuerung (110, 210, 310) dazu ausgelegt ist zu bewirken, dass die Blutdruckmessvorrichtung (130, 230, 330) eine erste Blutdruckmessung des Patienten als Reaktion darauf durchführt, dass ein erster geschätzter Blutdruck von einer ersten Basislinie um einen ersten Schwellenbetrag abweicht, wobei die Steuerung (110, 210, 310) dazu ausgelegt ist zu bewirken, dass die Blutdruckmessvorrichtung (130, 230, 330) eine zweite Blutdruckmessung (130, 230, 330) des Patienten als Reaktion darauf durchführt, dass ein zweiter geschätzter Blutdruck von einer zweiten Basislinie, die von der ersten Basislinie verschieden ist, um einen zweiten Schwellenbetrag abweicht,
**dadurch gekennzeichnet, dass**
die Steuerung (110, 210, 310) dazu ausgelegt ist, den ersten Schwellenbetrag basierend auf einer Differenz zwischen der ersten Basislinie und einer Alarmgrenze zu erhalten und den zweiten Schwellenbetrag basierend auf einer Differenz zwischen der zweiten Basislinie und einer Alarmgrenze zu erhalten; und
einen Patientenstatusdetektor (140, 240, 340), der operativ mit der Steuerung gekoppelt ist, wobei der Patientenstatusdetektor (140, 240, 340) einen Beschleunigungsmesser umfasst, der dazu ausgelegt ist zu bestimmen, ob der Patient liegt und ob sich der Patient nicht bewegt;
wobei die Steuerung dazu ausgelegt ist zu bewirken, dass die Blutdruckmessvorrichtung eine Blutdruckmessung des Patienten nur dann durchführt, wenn der Patientenstatusdetektor anzeigt, dass der Patient nicht hochgelagert ist und sich der Patient nicht zu stark bewegt, um die Messung zu beeinflussen.

2. Vorrichtung nach Anspruch 1, wobei die Steuerung (110, 210, 310) dazu ausgelegt ist zu verhindern, dass die Blutdruckmessvorrichtung (130, 230, 330) die zweite Blutdruckmessung durchführt, bis eine Zeitspanne seit der ersten Blutdruckmessung des Patienten abgelaufen ist.

3. Vorrichtung nach einem der Ansprüche 1-2, wobei die erste Basislinie basierend auf einem gemessenen Blutdruck des Patienten eingestellt wird.

4. Vorrichtung nach einem der Ansprüche 1-3, wobei der Monitor (120, 220, 320) dazu ausgelegt ist, einen Aortenblutdruck des Patienten zu schätzen.

5. Einrichtung nach einem der Ansprüche 1-3, wobei der Monitor (120, 220, 320) ein Pulsoximeter ist.

6. Vorrichtung nach einem der Ansprüche 1-5, wobei die Blutdruckmessvorrichtung (130, 230, 330) eine aufblasbare Manschette aufweist.

7. Vorrichtung nach einem der Ansprüche 1-6, wobei die Steuerung (110, 210, 310) dazu ausgelegt ist zu bewirken, dass die Blutdruckmessvorrichtung die erste Blutdruckmessung des Patienten als Reaktion darauf durchführt, dass der erste geschätzte Blutdruck von der ersten Basislinie um einen Betrag abweicht, der größer als ein Schwellenbetrag ist.

8. Einrichtung nach einem der Ansprüche 1-7, ferner umfassend:
eine Eingangsschnittstelle (214), die dazu ausgelegt ist, ein Signal zu empfangen, das indikativ für eine Blutdruckschätzung eines Patienten ist;
eine Ausgangsschnittstelle (212), die dazu ausgelegt ist, ein Signal bereitzustellen, um eine erste Blutdruckmessung des Patienten als Reaktion auf das Empfangen eines Signals durchzuführen, das anzeigt, dass ein geschätzter Blutdruck des Patienten von einer Basislinie abweicht; und
eine Steuerung (110, 210, 310), die mit der Eingangsschnittstelle (214) und der Ausgangsschnittstelle (212) gekoppelt ist und dazu ausgelegt ist, eine zweite Blutdruckmessung des Patienten zu verhindern, bis eine Zeitspanne seit der ersten Blutdruckmessung des Patienten abgelaufen ist.

9. Einrichtung nach Anspruch 8, wobei die Eingangsschnittstelle (214) dazu ausgelegt ist, operativ mit einem Pulsoximeter gekoppelt zu werden.

10. Vorrichtung nach einem der Ansprüche 8-9, wobei die Ausgangsschnittstelle (212) dazu ausgelegt ist, operativ mit einer nicht-invasiven Blutdruckmessvorrichtung (130, 230, 330) gekoppelt zu werden.

11. Einrichtung nach einem der Ansprüche 8-9, wobei die Ausgangsschnittstelle (212) dazu ausgelegt ist, operativ mit einer Blutdruckmanschette gekoppelt zu werden.

12. Einrichtung nach Anspruch 8, ferner umfassend:
einen Patientenstatusdetektor (140, 240, 340), der operativ mit der Steuerung gekoppelt ist.

## Revendications

1. Appareil (100, 200, 300), comprenant :
un moniteur (120, 220, 320) configuré pour estimer une pression artérielle d'un patient ;
un dispositif de mesure de la pression artérielle (130, 230, 330) configuré pour mesurer une pression artérielle du patient ;
un contrôleur (110, 210, 310) couplé fonctionnellement au moniteur (120, 220, 320) et au dispositif de mesure de la pression artérielle (130, 230, 330), le contrôleur (110, 210, 310) étant configuré pour amener le dispositif de mesure de la pression artérielle (130, 230, 330) à prendre une première mesure de la pression artérielle du patient en réponse à une première la pression artérielle estimée s'écartant d'une première valeur seuil par rapport à une première ligne de base, le contrôleur (110, 210, 310) étant configuré pour amener le dispositif de mesure de la pression artérielle (130, 230, 330) à prendre une seconde mesure de la pression artérielle (130, 230, 330) du patient en réponse à une deuxième pression artérielle estimée s'écartant d'une deuxième valeur seuil par rapport à une deuxième ligne de base différente de la première ligne de base,
**caractérisée en ce que**
le contrôleur (110, 210, 310) est configuré pour obtenir la première valeur seuil sur la base d'une différence entre la première ligne de base et une limite d'alarme, et pour obtenir la seconde valeur seuil sur la base d'une différence entre la seconde ligne de base et une limite d'alarme ; et
un détecteur d'état de patient (140, 240, 340) couplé de manière fonctionnelle au contrôleur, le détecteur d'état de patient (140, 240, 340) comprenant un accéléromètre configuré pour déterminer si le patient est allongé et si le patient ne se déplace pas ;
le contrôleur étant configuré pour amener le dispositif de mesure de la pression artérielle à prendre une mesure de la pression artérielle du patient uniquement si le détecteur d'état du patient indique que le patient n'est pas en position surélevée et que le patient ne bouge pas trop au point d'affecter la mesure.

2. Appareil selon la revendication 1, dans lequel le contrôleur (110, 210, 310) est configuré pour empêcher le dispositif de mesure de la pression artérielle (130, 230, 330) de prendre la seconde mesure de pression artérielle jusqu'à ce qu'une période de temps ait expiré à partir de la première mesure de pression artérielle du patient.

3. Appareil selon l'une quelconque des revendications 1 et 2, dans lequel la première ligne de base est établie sur la base d'une pression artérielle mesurée du patient.

4. Appareil selon l'une quelconque des revendications 1 à 3, dans lequel le moniteur (120, 220, 320) est configuré pour estimer une pression artérielle aortique du patient.

5. Appareil selon l'une quelconque des revendications 1 à 3, dans lequel le moniteur (120, 220, 320) est un oxymètre à impulsions.

6. Appareil selon l'une quelconque des revendications 1 à 5, dans lequel le dispositif de mesure de la pression artérielle (130, 230, 330) comprend un brassard gonflable.

7. Appareil selon l'une quelconque des revendications 1 à 6, dans lequel le contrôleur (110, 210, 310) est configuré pour amener le dispositif de mesure de la pression artérielle à prendre la première mesure de la pression artérielle du patient en réponse à la première pression artérielle estimée s'écartant d'une quantité supérieure à une valeur seuil par rapport à la première ligne de base.

8. Appareil selon l'une quelconque des revendications 1 à 7, comprenant en outre :
une interface d'entrée (214) configurée pour recevoir un signal indicatif d'une estimation de la pression artérielle d'un patient ;
une interface de sortie (212) configurée pour fournir un signal pour effectuer une première mesure de pression artérielle du patient en réponse à la réception d'un signal indiquant qu'une pression artérielle estimée du patient s'écarte d'une ligne de base ; et
un contrôleur (110, 210, 310) couplé à l'interface d'entrée (214) et à l'interface de sortie (212) et configuré pour empêcher une seconde mesure de pression artérielle du patient jusqu'à ce qu'une période de temps soit écoulée à partir de la première mesure de pression artérielle du patient.

9. Appareil selon la revendication 8, dans lequel l'interface d'entrée (214) est configurée pour être couplée fonctionnellement à un oxymètre à impulsions.

10. Appareil selon l'une quelconque des revendications 8 à 9, dans lequel l'interface de sortie (212) est configurée pour être couplée fonctionnellement à un dispositif de mesure de pression artérielle non invasif (130, 230, 330).

11. Appareil selon l'une quelconque des revendications 8 à 9, dans lequel l'interface de sortie (212) est configurée pour être couplée fonctionnellement à un brassard de pression artérielle.

12. Appareil selon la revendication 8, comprenant en outre :
un détecteur d'état de patient (140, 240, 340) couplé fonctionnellement au contrôleur.
